(19) Europäisches Patentamt
European Patent Office
Office européen des brevets

(11) Publication number: **0 154 296**
**B1**

# (12) EUROPEAN PATENT SPECIFICATION

(45) Date of publication of patent specification: **05.09.90**

(21) Application number: **85102220.2**

(22) Date of filing: **28.02.85**

(51) Int. Cl.[5]: **A 61 K 7/06**, A 61 K 31/10, A 61 K 31/105, A 61 K 31/27, A 61 K 31/40, A 61 K 31/44, A 61 K 31/445, A 61 K 31/48

## (54) Hair growth stimulating agents and their use.

(30) Priority: **07.03.84 GB 8405896**

(43) Date of publication of application:
**11.09.85 Bulletin 85/37**

(45) Publication of the grant of the patent:
**05.09.90 Bulletin 90/36**

(84) Designated Contracting States:
**AT BE CH DE FR GB IT LI LU NL SE**

(56) References cited:
**EP-A-0 003 286**
**EP-A-0 058 134**
**EP-A-0 159 519**
**DE-A-3 104 703**
**DE-A-3 118 128**
**DE-B-1 149 858**
**FR-A- 5 916**
**GB-A-1 563 824**
**GB-A-2 083 036**
**GB-A-2 111 053**
**US-A-2 719 813**
**US-A-4 005 211**
**US-A-4 246 263**

**M. NEGWER "Organisch-Chemische Arzneimittel und ihre Synonyma" fifth edition, vol. II, 1978; AKADEMIE-VERLAG, Berlin, page 1253**

(73) Proprietor: **SANDOZ AG**
**Lichtstrasse 35**
**CH-4002 Basel (CH)**
(84) **BE CH**

(73) Proprietor: **SANDOZ-PATENT-GMBH**
**Humboldtstrasse 3**
**D-7850 Lörrach (DE)**
(84) **DE**

(73) Proprietor: **SANDOZ-ERFINDUNGEN**
**Verwaltungsgesellschaft m.b.H.**
**Brunner Strasse 59**
**A-1235 Wien (AT)**
(84) **AT**

(72) Inventor: **Hiestand, Peter**
**Schönenbuchstrasse 13A**
**CH-4123 Allschwil (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 154 296 B1

Courier Press, Leamington Spa, England.

## Description

The present invention relates to a new use for prolactin secretion stimulating agents, in particular to their use as hair growth stimulating agents, as well as to novel compositions comprising prolactin secretion stimulating agents, for use in the stimulation of hair growth.

In accordance with the present invention it has now surprisingly been found that prolactin secretion stimulating agents can be employed to stimulate hair growth, i.e. to stimulate hair formation by dermal hair follicles. In particular it has been found that prolactin secretion stimulating agents stimulate hair growth in cases where diminution or failure of follicular hair growth, including hair loss, is due to ageing, as well as in cases where diminution or failure of follicular hair growth, including hair loss, is morbid or disease-related, e.g. consequential to disease, e.g. infection or, in particular, disease having an aetiology comprising disturbance of the immune system. Prolactin secretion stimulating agents can thus be employed in accordance with the method of the invention hereinafter defined, either cosmetically or as an adjunct to medical treatment, e.g. for counteracting hair loss or baldness, e.g. pattern baldness, consequential to the natural process of ageing (and whether or not the onset of baldness is "premature"), as well as for the treatment of disease-related hair loss or baldness.

Prolactin secretion stimulating agents which may be employed in accordance with the present invention may belong to any of the various classes of compound known in the art capable of increasing prolactin levels within the general body tissue, e.g. by stimulation of prolactin release from the adenohypophysis, including ergot alkaloids, neuroleptic agents and anti-emetic agents having prolactin secretion stimulating activity.

A group of prolactin secretion stimulating agents which have been found to be especially useful as stimulators of hair growth are those compounds which, in addition to stimulating prolactin secretion, are effective as immunostimulants, e.g. which effect *in vivo* stimulation of both the primary and secondary immuno response. Of particular interest within this group are immunostimulants belonging to the class of mono-, di- and tri-sulfide compounds, for example of the various types disclosed *inter al.* in DE—A—31 18 128; GB—A—2 083 036 and GB—A—2 111 053, European Patent No. 0 058 134 and U.S. Patent No. 4 246 263.

Preferred prolactin secretion stimulating agents for use in accordance with the present invention and belonging to this latter group are: hydroxyalkyl-, hydroxycycloalkyl-, alkoxyalkyl-, alkoxycycloalkyl-, aminoalkyl, aminocycloalkyl- and epoxyalkyl- -mono-, -di- and -trisulfides, as well as esters and carbamates of such hydroxyalkyl- and -hydroxycycloalkyl- -mono-, -di- and trisulfides; in particular, hydroxyalkyl- and hydroxycycloalkyl- -mono-, -di- and -trisulfides, and esters and carbamates thereof; and especially ($C_{2-3}$hydroxyalkyl)-mono-, -di- and -trisulfides, and esters and carbamates thereof.

Especially preferred mono-sulfides as defined above are the compounds of the aforementioned U.S. Patent No. 4 246 263, in particular the compound 2-hydroxyethyl 4-pyridylmethyl sulfide of formula

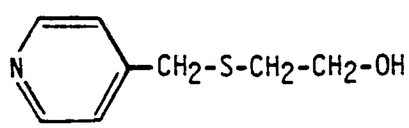

also known and referred to herein as RISTIANOL, as well as the compound 2-mercapto-ethanol [HS—$CH_2$—$CH_2$—OH: herein "COMPOUND B"].

Di and -tri-sulfides as defined above are for example described together with their use as immunostimulants, in the aforementioned European Patent No. 0 058 134, and GB—A—2 111 053, and preferred sub-groups of di- and tri-sulfides for use in accordance with the present invention are as defined therein.

An especially preferred sub-group defined in GB—A—2 111 053 for use in accordance with the present invention comprises the di- and tri-sulfides of formula I

$$R_1—(S)_x—R_2 \qquad (I)$$

wherein

$R_1$ is hydroxyalkyl, hydroxycycloalkyl, alkoxyalkyl, alkoxycycloalkyl, aminoalkyl, aminocycloalkyl or epoxyalkyl (especially hydroxyalkyl wherein the hydroxy group is on the 2-position or is terminal, 2-hydroxy-cycloalkyl, 2-alkoxyalkyl, 2-alkoxycycloalkyl, 2-aminoalkyl or 2-epoxyalkyl),

$R_2$ is alkyl or cycloalkyl or has the meanings given for $R_1$ above, and

X is 2 or 3;

as well as the esters and carbamates of such di- or tri-sulfides wherein $R_1$ and/or $R_2$ is hydroxyalkyl or hydroxycycloalkyl;

and, most especially, di- and tri-sulfides of formula I wherein $R_1$ and $R_2$ are, independently, hydroxyalkyl and, in particular, wherein $R_1$ and $R_2$ are, independently, $C_{2-3}$ hydroxyalkyl.

Particular compounds disclosed in GB—A—2 111 053 suitable for use in accordance with the present invention are:

COMPOUND C: $HO-(CH_2)_2-S-S-(CH_2)_2-OH$.

COMPOUND D: $HO-(CH_2)_3-S-S-(CH_2)_3-OH$.

COMPOUND E:
$$\begin{array}{l} \quad\quad\quad\ \ OH \quad\quad\quad\quad\quad\quad\quad\quad\ OH \\ Cl-CH_2-\overset{|}{C}H-CH_2-S-S-CH_2-\overset{|}{C}H-CH_2-Cl. \end{array}$$

COMPOUND F: $CH_3O-(CH_2)_2-S-S-(CH_2)_2-OCH_3$.

COMPOUND G: 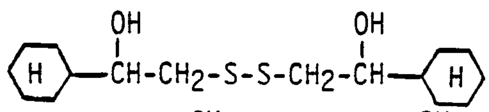

COMPOUND H:
$$\begin{array}{l} \quad\quad\quad\quad\quad\quad\quad OH \quad\quad\quad\quad\quad\quad\quad\quad\ OH \\ CH_3-(CH_2)_3-\overset{|}{C}H-CH_2S-S-CH_2-\overset{|}{C}H-(CH_2)_3-CH_3. \end{array}$$

COMPOUND I:
$$\begin{array}{l} \quad\quad\ \ OH \quad\quad\quad\quad\quad\quad\quad\quad\ OH \\ CH_3-\overset{|}{C}H-CH_2-S-S-CH_2-\overset{|}{C}H-CH_3. \end{array}$$

COMPOUND J:
$$\begin{array}{l} \quad\quad\quad\quad\ OH \quad\quad\quad\quad\quad\quad\quad\quad\ OH \\ HO-CH_2-\overset{|}{C}H-CH_2-S-S-CH_2-\overset{|}{C}H-CH_2-OH. \end{array}$$

COMPOUND K: $CH_3O-CH_2-C(CH_3)_2-S-S-C(CH_3)_2-CH_2-OH$.

COMPOUND L: 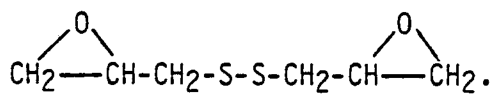

COMPOUND M: $CH_3-NH-(CH_2)_2-S-S-(CH_2)_2-NH-CH_3$.

COMPOUND N: $i-C_3H_7-S-S-(CH_2)_2-OH$.

COMPOUND O: $t-C_4H_9-S-S-(CH_2)_2-OH$.

COMPOUND P:
$$\begin{array}{l} \quad\quad\quad\quad\quad\quad\ CH_3 \quad\quad\quad\quad\quad\quad\quad\ OH \\ CH_3-CH_2-\overset{|}{C}H--S-S-CH_2-\overset{|}{C}H-CH_3. \end{array}$$

COMPOUND Q:
$$\begin{array}{l} \quad\quad\quad\quad\quad\quad\quad\quad\quad\quad\quad\quad OH \\ i-C_3H_7-S-S-CH_2-\overset{|}{C}H-CH_3. \end{array}$$

COMPOUND R:
$$\begin{array}{l} \quad\quad\quad\quad\quad\quad\quad\quad\quad\quad\quad\quad OH \\ t-C_4H_9-S-S-CH_2-\overset{|}{C}H-CH_3. \end{array}$$

COMPOUND S:
$$\begin{array}{l} \quad\quad\quad\quad\quad\quad\ CH_3 \\ CH_3-CH_2-\overset{|}{C}H-S-S-(CH_2)_2-OH. \end{array}$$

COMPOUND T: $i-C_3H_7-CH(i-C_3H_7)-S-S-(CH_2)_2-OH$.

COMPOUND U: $CH_3-NH-CO-O-(CH_2)_2-S-S-S-(CH_2)_2-O-CO-NH-CH_3$.

COMPOUND V: 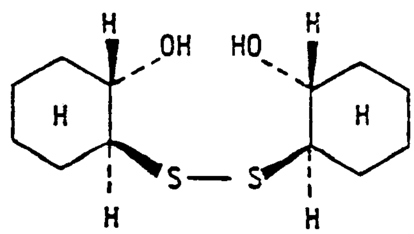 (Racemate), and

COMPOUND W: 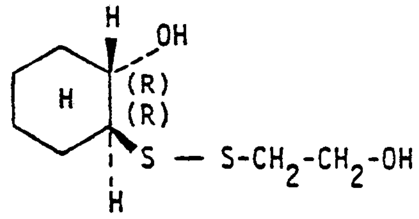 (Racemate)

Of the above group of compounds (C to W), COMPOUND C (2,2'-dithioethanol) is especially preferred.

Most preferred for use in accordance with the present invention are the di-sulfides disclosed in the aforementioned European Patent No. 0 058 134 of formula II

$$R_3O-(CH_2)_m-S\left[-S-(CH_2)_p-S-\right]_y S-(CH_2)_n-OR_4 \qquad (II)$$

wherein

$R_3$ and $R_4$ are, independently, hydrogen or $C_{1-5}$alkyl,
m and n are, independently, 2 or 3
p is an integer from 2 to 6, and
y is 1, 2 or 3,

whereby when y is 2 or 3, each p may be the same or different, as well as esters of said compounds wherein $R_3$ and/or $R_4$ is hydrogen.

Alkyl groups as $R_3$ and $R_4$ in formula II may be branched or, preferably, straight-chain. Preferably they contain 1 to 4 carbon atoms, methyl being the most preferred. Most preferably $R_3$ and $R_4$ are the same, e.g. are both hydrogen. m and n are also preferably the same and are most preferably 2. When y is 2 or 3, each p may be different, preferably each p is the same, and more preferably each p is 2. Most conveniently y is 1. Especially preferred are compounds of formula I, wherein y is 1 and p is 2.

The most preferred compound for use in accordance with the invention is the compound S,S'-ethylene-bis-(2-dithioethanol) [formula II, $R_3$ and $R_4$ both = H; m, n and p all = 2; y = 1: herein "COMPOUND X"].

Effectiveness of prolactin secretion stimulating agents as hair growth stimulants can be shown in animal models, as well as in trials in humans, for example in accordance with the following methods:

I: Stimulation of hair growth in ageing mice

Female mice lose back fur as part of the normal process of ageing. Groups of 5 to 15, healthy female mice ($C_{57}B1$) are kept under standard laboratory conditions until extensive or total dorsal hair loss has occurred (generally at ca. 40—45 weeks of age). Test substance, e.g. COMPOUND X, is thereafter applied to said mice orally at varying dosages/group, the mice in each group receiving test substance at constant dosage, 2 × weekly for 3 weeks. During the course of the test the mice are allowed access to food and water *ad libitum.* At the end of four weeks all test animals are examined and the condition of dorsal hair growth recorded.

In the above test, regeneration of dorsal hair is observed in animals employing prolactin secretion stimulating agents as test substance at dosages sufficient to effect stimulation of basal prolactin secretion levels.

Thus in the case of mono-, di- and tri-sulfides as hereinbefore set forth, significant stimulation of dorsal hair growth is recorded on oral administration at dosages of from 0.1 to 10 mg/kg/dose, administered in accordance with the above indicated regimen. Especially good results are obtained employing COMPOUND X, for which significant stimulation of dorsal hair growth is recorded at dosages of from 0.01 to 10 mg/kg/dose with the majority of mice exhibiting no signs of baldness at the end of three weeks on administration at dosages of from 1.0 mg/kg/dose upwards.

Similar results are obtained on administration of prolactin secretion stimulating agents of other classes as hereinbefore described.

II and III: Stimulation of hair growth in humans

*II. Subjects exhibiting disease-related hair loss*

Test substance, e.g. COMPOUND X, is applied topically to the scalp of volunteers exhibiting hair loss consequent to Hashimoto's disease. The scalps of individual volunteers are examined prior to commencement of the trial and scalp condition is recorded.

For the purposes of application, the test substance is put up in lanolin, e.g. as described hereinafter in example 3, to give an ointment comprising 1% (w/w) of test substance. Ointment is applied topically to one side of the scalp only of each volunteer. Application is performed 1 × daily throughout the trial period and is distributed with light massage. The condition of the scalp is examined daily and hair growth on the treated half compared with growth on the untreated half.

In this trial stimulation of hair growth on the treated half of the scalp as compared with the untreated half is recorded on application of ointments comprising a prolactin secretion stimulating agent as active ingredient. Thus on daily application of 0.5 g of ointment containing COMPOUND X as active ingredient (= 0.005 g COMPOUND X/half-scalp/day) stimulation of hair growth on the treated half of the scalp as compared with the untreated half is recorded after ca. 2 weeks of treatment. After 5 and more weeks of treatment, stimulation of hair growth on the treated half is advanced and hair loss or baldness significantly reduced. No or substantially no change in hair condition is recorded on the untreated side.

*III. Subjects exhibiting age-related hair loss (i)*

The methodology of test II is repeated but employing volunteers exhibiting scalp hair loss or baldness consequent to ageing without substantial loss of scalp hair follicles. (The medical history of all volunteers is thoroughly checked prior to commencement of the trial and all volunteers are subjected to medical control to ensure that hair loss is not disease related. In addition the scalp of each volunteer is examined to ensure that hair loss is unaccompanied by substantial depletion of scalp hair follicles, subjects exhibiting extensive hair follicle loss being excluded).

Results obtained are analogous to those obtained in Trial II above. Thus on application of e.g. COMPOUND X in ointment form at a daily dosage rate as for Trial II, stimulation of hair growth on the treated half of the scalp as compared with the untreated half is recorded after ca. 2 to 4 weeks of treatment. After 5 and more weeks stimulation of hair growth in the treated area is advanced and hair loss or baldness reduced. No, or substantially no change in hair growth in recorded on the untreated side.

*IV. Subjects exhibiting age related hair loss (ii)*

The methodology of test III is repeated. In this case however trial subjects are divided into two groups, one group receiving test substance either topically or systemically, e.g. orally and the other group receiving placebo. At the end of the trial period (ca. 2 to 4 weeks) scalp hair growth in the two trial groups is compared.

In this trial stimulation of scalp hair growth in subjects belonging to the trial group receiving test substance, e.g. COMPOUND X topically in accordance with the regimen described in trial II or orally at dosages of from 5 to 200 mg 2 × weekly, is observed as compared with subjects in the trial group receiving placebo.

Comparable results to those obtained with COMPOUND X are obtainable in Trials II, III and IV above on administration of other prolactin stimulating agents, in particular RISTIANOL and COMPOUNDS A through W at the same or equivalent dosage rates.

The present invention accordingly provides: the use of a prolactin stimulating agent for the manufacture of a composition for use in the stimulation of hair growth.

In practicing the method of the present invention, the prolactin secretion stimulating agent may be administered e.g. either systemically, for example orally, or topically, i.e. dermally. However since it will be more commonly desired to stimulate hair growth at a particular site on the body, for example the scalp or part of the scalp, and targeting is more readily effected via topical application, topical application is generally preferred. Moreover topical administration will generally have the advantage of avoiding or reducing likely occurrence of unwanted side-reactions, e.g., resultant from the prolactin secretion stimulating agent's regular pharmaceutical utility or utilities.

It will be appreciated that where compounds are to be administered other than topically, e.g. orally, they must necessarily be pharmaceutically acceptable, i.e. physiologically tolerable at the dosages administered. Where compounds are to be administered topically they must necessarily be acceptable for topical application. Where compounds are to be administered other than topically, e.g. orally, and these contain groups or groupings which are susceptible to hydrolysis *in vivo*, i.e. contain physiologically hydrolysable groupings, as in the case of esters and carbamates as hereinbefore described, these groups or groupings must also be pharmaceutically acceptable, i.e. give rise to metabolic products, e.g. acids and amides, which are themselves physiologically tolerable at desired dosage levels. Prolactin secretion stimulating agents for use in the method of the invention containing one or more acidic or basic groups and which also exist in salt form, e.g. in acid addition salt form, may also be employed either in free or in salt form. Where non-topical administration, e.g. oral administration is contemplated, salts used will be pharmaceutically acceptable salts. When topical administration is contemplated, salt forms used will be acceptable for topical application. Such salt forms are known from the literature and generally have the same or similar order of activity to the free forms. Throughout the present specification and claims references to prolactin secretion stimulating agents as well as to individual compounds and to classes of prolactin secretion stimulating agent are to be interpreted accordingly and are to be understood as including such salt forms as aforesaid where appropriate.

Dosage rates to be employed in practicing the method of the present invention will of course vary, depending on a variety of factors including e.g. the particular prolactin secretion stimulating agent employed, the form in which it is employed, the mode of administration (in particular whether administration is topical or otherwise) the specific condition to be treated (e.g. whether morbid, disease related or age related), as well as the effect desired.

Where administration is non-topical, e.g. systemic, for example oral, dosages will generally be of an

order sufficient, as known in the art, to effect prolactin secretion stimulation, e.g. to effect elevation of basal prolactin serum levels. Appropriate dosage rates will accordingly be e.g. of similar order or slightly less than those required on administration of the same prolactin secretion stimulating agent via the same route for other known indications.

In the case of mono-, di- and tri-sulfide compounds as hereinbefore defined, e.g. RISTIANOL and, in particular, COMPOUNDS B through W, and especially COMPOUND X, suitable oral dosage rates for use in practicing the method of the present invention are of the order of from 0.01 to 10 mg/kg administered 2 × weekly or of from 0.005 to 3 mg/kg administered daily. Suitable oral dosage forms for stimulating hair growth in humans accordingly comprise from 0.75 to 750 mg, e.g. to 500 mg (for bi-weekly application) or from 0.4 to 200 mg (for daily application) mono-, di- or tri-sulfide, e.g. COMPOUND X, together with one or more pharmaceutically acceptable diluents or carriers therefor.

Suitable oral dosage forms include e.g. tablets and capsules as well as liquid dosage forms, e.g. solutions, emulsions and dispersions. These may be prepared in conventional manner, e.g. as described in the examples hereinafter provided.

As previously noted however, in practicing the method of the present invention, the active ingredient, i.e. selected prolactin secretion stimulating agent, is preferably administered topically. In the case of mono-, di- and tri-sulfides as hereinbefore defined, e.g. RISTIANOL and compounds C through X inclusive, topical application is in particular preferred.

Appropriateness of topical administration is of course dictated by the novel use (use in hair growth stimulation) provided by the present invention. In that compositions for topical application comprising prolactin secretion stimulating agents as active ingredient are novel and inherent to the novel use for prolactin secretion stimulating agents herein described and claimed, in a further embodiment the present invention also provides: A composition for use in the stimulation of hair growth, said composition being formulated or presented so as to provide for, or to permit, convenient dermal application and comprising a prolactin secretion stimulating agent as active component, together with one or more topically applicable diluents or carriers therefor.

Preferred prolactin secretion stimulating agents for use in the novel compositions of the invention are as hereinbefore set forth in relation to hair growth stimulation use.

Compositions for dermal application in accordance with the present invention may be liquid or solid. Suitable liquid compositions include e.g. solutions, emulsions or dispersions. Suitable solid compositions include e.g. powders and dermally applicable films, e.g. comprising the active ingredient retained within a permeable polymeric film material.

In accordance with the invention said compositions are formulated or presented so as to provide for or to permit convenient topical application, e.g. formulated or presented in such a manner as to permit convenient application of active agent (i.e. prolactin secretion stimulating agent) to predetermined areas of the skin, in predetermined amounts, e.g. without undue loss of active ingredient applied and/or without undue inconvenience to the subject to be treated.

Various forms and systems suitable for the application of active agents topically are known in the art and any appropriate known form or system may be employed in relation to the present invention.

Liquid compositions formulated in accordance with the present invention may be free-flowing or semi-solid. Free-flowing forms include, e.g. topically applicable lotions and sprays. As known in the art, such free-flowing forms will generally comprise a diluent or carrier medium susceptible to rapid evaporation such that the active ingredient remains entirely or substantially entirely at the site of application.

Preferably liquid compositions formulated in accordance will be semi-solid, e.g. be of relatively high viscosity, such that they are distributable over the surface to which they are to be applied but which do not readily spread or run of their own accord. Such forms include e.g. ointments, gels, pastes and creams. Suitably such semi-solid compositions will comprise a diluent or carrier medium component susceptible to rapid evaporation, to assist retention of the composition at the site of application.

Suitable diluent or carrier medium components susceptible to evaporation, for use in the preparation of liquid compositions, including semi-solid compositions, as described above, are in particular topically applicable alcohols volatile at room temperature, for example $C_{1-4}$alkanols such as ethanol and isopropanol.

Solid compositions formulated in accordance with the present invention are in particular, dermally applicable powders.

Alternatively compositions in accordance with the present invention may be presented in such a manner so as to provide for, or to permit, convenient dermal application, e.g. may form the effective component of a dermal application device, such as a plaster, dermal patch or cataplasm.

Most preferably, compositions in accordance with the invention will be formulated or presented so as to provide for, or to permit, conventional application to the scalp.

As noted above, preferred compositions in accordance with the invention are semi-solid. Such forms suitably comprise an appropriate base material, e.g. an ointment base for example a hydrocarbon (oleaginous) base such as white petroleum, an absorption (anhydrous) base such as hydrophilic petrolatum, an emulsion (w/o type) base such as lanolin or cold cream, an emulsion (o/w type) base such as hydrophilic ointment, or a water soluble base such as polyethylene glycol ointment. Suitable base components include e.g. cholesterols, stearyl alcohols, white wax, and white petrolatum. Suitable

emulsifier systems in cream formulations may comprise a non-ionic, anionic, cationic or amphoteric emulsifier such as sodium lauryl sulfate, polyoxyethylene and polyoxypropylene fatty alcohol ethers, polyoxyethylene fatty acid esters, polyoxyethylene sorbitan fatty acid esters, polyoxyethylene glycol fatty acid esters, polyol fatty acid esters such as glyceryl monostearate and ethoxylated lanolin derivatives.

Topical formulations in accordance with the invention may also contain any of the commonly employed preserving agents such as methyl paraben, propyl paraben, benzyl alcohol, sorbic acid or quaternary ammonium compounds. They may also, where appropriate, contain humectants or skin penetration enhancers such as dimethyl sulfoxide, dimethylacetamide and dimethylformamide.

Amounts of active ingredient (i.e. prolactin secretion inhibiting agent) for use in topical composition may of course vary considerably e.g. in accordance with the various parameters hereinbefore mentioned in relation to non-topical application, and in particular depending upon the particular application form employed and the intended frequency of application.

In the case of e.g. mono-, di- and tri-sulfide compounds as hereinbefore defined, e.g. RISTIANOL and, in particular COMPOUNDS B through W, and especially COMPOUND X, forms for topical application suitably contain from 0.01% to 95.0%, more suitably from 0.05 to 10%, especially 1.0% (w/w) of active ingredient. An effective application rate for topical application of such compounds e.g. of COMPOUND X, is of the order of from 1.0 to 0.01 preferably from 0.5 to 0.05, e.g. 0.1 mg/cm$^2$ skin/day, and forms for topical administration are appropriately formulated and contain the compound at such a concentration as to permit application at such rate.

The following examples are illustrative of compositions suitable for use in accordance with the method of the invention:

Example 1

Production of solid compositions for oral application

Tablets may contain the active agent in admixture with conventional pharmaceutically acceptable excipients, e.g. inert diluents, such as calcium carbonate, sodium carbonate, lactose and talc, granulating and disintegrating agents, e.g. starch and alginic acid, flavouring, colouring and sweetening agent, binding agents, e.g. starch, gelatin and acacia, and lubricating agents, e.g. magnesium stearate, stearic acid and talc. The tablets may be uncoated or coated by known techniques to delay disintegration and absorption in the gastrointestinal tract and thereby provide a sustained action over a long period.

For the manufacture of tablets, the active ingredient, can be mixed with lactose and granulated with water, 0.5% sodium alginate or 1% gelatine solution. The dried granulate is compressed into tablets in the presence of about 5% of talcum, 5% of corn starch and 0.1% of magnesium stearate. In this way, there are obtained, e.g. tablets of the following composition:

| | |
|---|---|
| Active ingredient, e.g. COMPOUND X | 20 mg |
| Lactose | 70 mg |
| Corn Starch | 5 mg |
| Talcum | 5 mg |
| Magnesium stearate | 0.1 mg |

Capsules may contain the active agent alone or admixed with an inert solid diluent, for example as mentioned above.

Capsules containing the ingredient indicated below may be prepared by conventional techniques.

| | |
|---|---|
| Active ingredient, e.g. COMPOUND X | 20 mg |
| Inert solid diluent (e.g. starch, caolin, calcium phosphate or carbonate, or lactose) | 20 mg |

Example 2

Production of liquid compositions, for oral application

Solutions, suspensions, emulsions, dispersions, syrups and elixirs may contain the active agent in admixture with any of the conventional excipients utilized for the preparation of such compositions, e.g. suspending agents (methylcellulose, tragacanth and sodium alginate), wetting agents (lecithin, polyoxyethylene stearate and polyoxyethylene sorbitan mono-oleate) flavouring, colouring and sweetening agents and preservatives (ethyl-*p*-hydroxybenzoate).

The following pharmaceutical composition is formulated with the indicated amount of active agent

using conventional techniques. The suspension is suitably administered 1 or 2 to 4 times per day depending on the full dosage required.

| | |
|---|---|
| Active ingredient | 20 |
| Sodium carboxy methyl cellulose U.S.P. | 12.5 |
| Flavour | q.s. |
| Colouring agent | q.s. |
| Methyl paraben, U.S.P. | 4.5 |
| Propyl paraben, U.S.P. | 1.0 |
| Polysorbate 80 (e.g. Tween 80), U.S.P. | 5.0 |
| Sorbitol solution, 70%, U.S.P. | 2.500 |
| Buffer agent to adjust pH for desired stability | q.s. |
| Water | q.s. to 5 ml |

Example 3
Production of a composition suitable for topical application

| | |
|---|---|
| Active ingredient, e.g. COMPOUND X | 1 g |
| Lanolin | 99 g |

The lanolin is liquified at 38 to 45°C, the active ingredient dissolves therein, and the whole is allowed to cool. The obtained ointment is used directly for topical application of 0.01 g active ingredient to the scalp/day = an application rate of 0.1 mg/cm$^2$/day.

Example 4
Compositions suitable for topical application

Compositions for topical application may also be prepared employing the ingredients indicated below. The compositions are formulated in conventional manner, and may contain as additional additives conventional stabilisers, preserving agents, anti-oxidants, etc. . . . as required.

| | |
|---|---|
| 4a. Active ingredient, e.g. COMPOUND X | 1 g |
| Aerosil® 200 | 8 g |
| Miglyol® 812 | 30 g |
| Ethanol (94%) to an end weight of | 100 g |
| 4b. Active ingredient, e.g. COMPOUND X | 1 g |
| Miglyol® 812 | 30 g |
| Ethylcellulose | 12 g |
| Ethanol (absolute) to an end weight of | 100 g |

4c. As example 4a, but employing isopropanol in place of ethanol.

4d. As example 4b, but employing isopropanol in place of ethanol.

| | |
|---|---|
| 4e. Active ingredient, e.g. COMPOUND X | 1 g |
| Aerosil® 200 | 8 g |
| Miglyol® 812 | 50 g |
| Ethanol (94%) to an end weight of | 100 g |

| | |
|---|---|
| 4f Active ingredient, e.g. COMPOUND X | 1 g |
| Miglyol® 812 | 50 g |
| Ethylcellulose | 12 g |
| Ethanol (absolute) to an end weight of | 100 g |

4g. As example 4a, but with inclusion of 5 g PEG 300 or 5 g propylene glycol.

4h. As example 4b, but with inclusion of 5 g PEG 300 or 5 g propylene glycol.

| | |
|---|---|
| 4i. Active ingredient, e.g. COMPOUND X | 1 g |
| Ethanol (94%) | 50 g |
| Polyvinylpyridolidone | 30 g |
| $H_2O$ (distilled) to an end weight of | 100 g |

| | |
|---|---|
| 4j. Active ingredient, e.g. COMPOUND X | 1 g |
| Ethanol (94%) | 50 g |
| Carbopol® 934 | 1.5 g |
| Diisopropanolamine | 1.5 g |
| $H_2O$ (distilled) to an end weight of | 100 g |

[Miglyol® 812 = a fractionated coconut oil product available from Dynamit Nobel AG — c.f. H. P. Fiedler, "Lexicon der Hilfsstoffe", 2nd. edition (hereinafter "F.-L.d.H.") p. 616.
Aerosil® 200 = a pure silica gel — c.f. F.-L.d.H., p. 94
PEG = polyethyleneglycol
Carbopol® 934 = an acrylic acid polymer available from W. Biesterfeld + Co. — c.f. F.-L.d.H., p. 206.]

**Claims for the Contracting States: BE CH DE FR GB IT LI LU NL SE**

1. Use of a hydroxyalkyl-, hydroxycycloalkyl-, alkoxyalkyl-, alkoxycycloalkyl-, aminoalkyl-, aminocycloalkyl- or epoxyalkyl- -mono-, -di- or -trisulfide; or ester or carbamate of a hydroxyalkyl- or hydroxycycloalkyl- -mono-, -di- or -trisulfide for the manufacture of a composition for use in the stimulation of hair growth.

2. Use according to claim 1, wherein the sulfide is a di- or trisulfide.

3. Use according to claim 1, wherein the sulfide is selected from 2-mercaptoethanol and 2-hydroxyethyl-4-pyridylmethyl sulfide.

4. Use according to claim 1, wherein the sulfide is 2,2'-dithioethanol.

5. Use according to claim 1, wherein the sulfide is a compound of formula II

$$R_3O\text{-}(CH_2)_m\text{-}S\left[S\text{-}(CH_2)_p\text{-}S\right]_y S\text{-}(CH_2)_n\text{-}OR_4 \qquad (II)$$

wherein:

$R_3$ and $R_4$ are, independently, hydrogen or $C_{1-5}$alkyl,

m and n are, independently, 2 or 3,
p is an integer from 2 to 6, and
y is 1, 2 or 3,
whereby when y is 2 or 3 each p may be the same or different, or ester of such compound wherein $R_3$ and/or $R_4$ is hydrogen.

6. Use according to claim 5 wherein the sulfide is S,S'-ethylene-bis-(2-dithioethanol).

**Claims for the Contracting State: AT**

1. A process for the preparation of a composition for use in the stimulation of hair growth which comprises admixing a hydroxyalkyl-, hydroxycycloalkyl-, alkoxyalkyl-, alkoxycycloalkyl-, aminoalkyl-, aminocycloalkyl- or epoxyalkyl- -mono-, -di- or -trisulfide; or ester or carbamate of a hydroxyalkyl- or hydroxycycloalkyl- -mono-, -di- or -trisulfide with a topically applicable diluent or carrier therefor.

2. A process according to claim 1, wherein the sulfide is a di- or -trisulfide.

3. A process according to claim 1 wherein the sulfide is selected from 2-mercaptoethanol and 2-hydroxyethyl-4-pyridylmethyl sulfide.

4. A process according to claim 1 wherein the sulfide is 2,2'-dithioethanol.

5. A process according to claim 1 wherein the sulfide is a compound of formula II

$$R_3O-(CH_2)_m-S\left[S-(CH_2)_p-S\right]_y S-(CH_2)_n-OR_4 \qquad (II)$$

wherein:
$R_3$ and $R_4$ are, independently, hydrogen or $C_{1-5}$alkyl,
m and n are, independently, 2 or 3,
p is an integer from 2 to 6, and
y is 1, 2 or 3,
whereby when y is 2 or 3 each p may be the same or different, or ester of such compound wherein $R_3$ and/or $R_4$ is hydrogen.

6. A process according to claim 5 wherein the sulfide is S,S'-ethylene-bis-(2-dithioethanol).

**Patentansprüche für die Vertragsstaaten: BE CH DE FR GB IT LI LU NL SE**

1. Verwendung eines Hydroxyalkyl-, Hydroxycycloalkyl-, Alkoxyalkyl-, Alkoxycycloalkyl-, Aminoalkyl-, Aminocycloalkyl- oder Epoxyalkyl-mono-, -di- oder -trisulfids oder eines Esters oder eines Carbamats eines Hydroxyalkyl- oder Hydroxycycloalkyl-mono-, -di- oder -trisulfids zur Herstellung einer Zusammensetzung zur Verwendung bei der Stimulation des Haarwachstums.

2. Verwendung nach Anspruch 1, wobei es sich bei dem Sulfid um ein Di- oder Trisulfid handelt.

3. Verwendung nach Anspruch 1, wobei das Sulfid unter 2-Mercaptoethanol und 2-Hydroxyethyl-4-pyridylmethylsulfid ausgewählt ist.

4. Verwendung nach Anspruch 1, wobei es sich bei dem Sulfid um 2,2'-Dithioethanol handelt.

5. Verwendung nach Anspruch 1, wobei es sich bei dem Sulfid um eine Verbindung der Formel II handelt

$$R_3O-(CH_2)_m-S\left[S-(CH_2)_p-S\right]_y S-(CH_2)_n-OR_4 \qquad (II)$$

worin:
$R_3$ und $R_4$ unabhängig voneinander Wasserstoff oder $C_{1-5}$Alkyl bedeuten,
m und n unabhängig voneinander den Wert 2 oder 3 haben,
p eine ganze Zahl mit einem Wert von 2 bis 6 ist und
y den Wert 1, 2 oder 3 hat,
wobei, wenn y den Wert 2 oder 3 hat, die einzelnen Symbole p gleich oder verschieden sein können, oder eines Esters einer derartigen Verbindung, in der $R_3$ und/oder $R_4$ Wasserstoff bedeuten.

6. Verwendung nach Anspruch 5, wobei es sich bei dem Sulfid um S,S'-Ethylen-bis-(2-dithioethanol) handelt.

**Patentansprüche für den Vertragsstaat: AT**

1. Verfahren zur Herstellung einer Zusammensetzung zur Verwendung bei der Stimulation des Haarwachstums, umfassend das Vermischen eines Hydroxyalkyl-, Hydroxycycloalkyl-, Alkoxyalkyl-, Alkoxycycloalkyl-, Aminoalkyl-, Aminocycloalkyl- oder Epoxyalkyl-mono-, -di- oder -trisulfids oder eines

Esters oder Carbamats eines Hydroxyalkyl- oder Hydroxycycloalkyl-mono-, -di- oder -trisulfids mit einem topisch anwendbaren Verdünnungsmittel oder Trägerstoff hierfür.

2. Verfahren nach Anspruch 1, wobei es sich bei dem Sulfid um ein Di- oder Trisulfid handelt.

3. Verfahren nach Anspruch 1, wobei das Sulfid unter 2-Mercaptoethanol und 2-Hydroxyethyl-4-pyridylmethylsulfid ausgewählt ist.

4. Verfahren nach Anspruch 1, wobei es sich bei dem Sulfid um 2,2'-Dithioethanol handelt.

5. Verfahren nach Anspruch 1, wobei es sich bei dem Sulfid um eine Verbindung der Formel II handelt

$$R_3O-(CH_2)_m-S\left[S-(CH_2)_p-S\right]_y S-(CH_2)_n-OR_4 \qquad (II)$$

worin:

$R_3$ und $R_4$ unabhängig voneinander Wasserstoff oder $C_{1-5}$Alkyl bedeuten,
m und n unabhängig voneinander den Wert 2 oder 3 haben,
p eine ganze Zahl mit einem Wert von 2 bis 6 ist und
y den Wert 1, 2 oder 3 hat,
wobei, wenn y den Wert 2 oder 3 hat, die einzelnen Symbole p gleich oder verschieden sein können, oder eines Esters einer derartigen Verbindung, in der $R_3$ und/oder $R_4$ Wasserstoff bedeuten.

6. Verfahren nach Anspruch 5, wobei es sich bei dem Sulfid um S,S'-Ethylen-bis-(2-dithioethanol) handelt.

**Revendications pour les Etats contractants: BE CH DE FR GB IT LI LU NL SE**

1. Utilisation d'un mono-, di- or trisulfure d'hydroxyalkyle, d'hydroxycycloalkyle, d'alcoxyalkyle, d'alcoxycycloalkyle, d'aminoalkyle, d'aminocycloalkyle ou d'époxyalkyle; ou d'un ester ou carbamate d'un mono-, di- ou trisulfure d'hydroxyalkyle ou d'hydroxycycloalkyle pour la préparation d'une composition destinée à l'utilisation dans la stimulation de la pousse des cheveux.

2. L'utilisation selon la revendication 1, caractérisée en ce que le sulfure est un di- ou trisulfure.

3. L'utilisation selon la revendication 1, caractérisée en ce que le sulfure est choisi parmi le 2-mercaptoéthanol et le sulfure de 2-hydroxyéthyle et de 4-pyridylméthyle.

4. L'utilisation selon la revendication 1, caractérisée en ce que le sulfure est le 2,2'-dithioéthanol.

5. L'utilisation selon la revendication 1, caractérisée en ce que le sulfure est un composé de formule II

$$R_3O-(CH_2)_m-S\left[S-(CH_2)_p-S\right]_y S-(CH_2)_n-OR_4 \qquad (II)$$

dans laquelle

$R_3$ et $R_4$ signifient, indépendamment, l'hydrogène ou un groupe alkyle en $C_1$—$C_5$,
m et n signifient, indépendamment, 2 ou 3,
p signifie un nombre entier de 2 à 6, et
y signifie 1, 2 ou 3,
chaque p pouvant être indentique ou différent lorsque y signifie 2 ou 3, ou un ester d'un tel composé dans lequel $R_3$ et/ou $R_4$ signifie l'hydrogène.

6. L'utilisation selon la revendication 5, caractérisée en ce que le sulfure est le S,S'-éthylène-bis-(2-dithioéthanol).

**Revendications pour l'Etat contractant: AT**

1. Un procédé de préparation d'une composition pour l'utilisation dans la stimulation de la pousse des cheveux, caractérisé en ce qu'il comprend le mélange d'un mono-, di- ou trisulfure d'hydroxyalkyle, d'hydroxycycloalkyle, d'alcoxyalkyle, d'alcoxycycloalkyle, d'aminoalkyle, d'aminocycloalkyle ou d'époxyalkyle; ou d'un ester ou carbamate d'un mono-, di- ou trisulfure d'hydroxyalkyle ou d'hydroxycycloalkyle avec un diluant ou un véhicule pour ce composé applicable par voie topique.

2. Un procédé selon la revendication 1, caractérisé en ce que le sulfure est un di- ou trisulfure.

3. Un procédé selon la revendication 1, caractérisé en ce que le sulfure est choisi parmi le 2-mercaptoéthanol et le sulfure de 2-hydroxyéthyle et de 4-pyridylméthyle.

4. Un procédé selon la revendication 1, caractérisé en ce que le sulfure est le 2,2'-dithioéthanol.

5. Un procédé selon la revendication 1, caractérisé en ce que le sulfure est un composé de formule II

$$R_3O-(CH_2)_m-S\left[S-(CH_2)_p-S\right]_y S-(CH_2)_n-OR_4 \qquad (II)$$

dans laquelle

$R_3$ et $R_4$ signifient, indépendamment, l'hydrogène ou un groupe alkyle en $C_1$—$C_5$,

m et n signifient, indépendamment, 2 ou 3,

p signifie un nombre entier de 2 à 6, et

y signifie 1, 2 ou 3,

chaque p pouvant être indentique ou différent lorsque y signifie 2 ou 3, ou un ester d'un tel composé dans lequel $R_3$ et/ou $R_4$ signifie l'hydrogène.

6. Un procédé selon la revendication 5, caractérisé en ce que le sulfure est le S,S'-éthylène-bis-(2-dithioéthanol).